# EUROPEAN PATENT SPECIFICATION

(11) **EP 4 058 130 B1**
(45) Date of publication and mention of the grant of the patent: **23.04.2025**
(21) Application number: 20885719.3
(22) Date of filing: 05.11.2020
(51) Int. Cl.: A61N 1/04, A61B 5/00, A61B 5/053, A61B 5/01, A61B 5/259, A61B 5/282, A61B 5/308, A61B 5/366, A61B 5/363

(54) **STRETCHABLE ELECTROCARDIOGRAM (ECG) APPARATUSES**
DEHNBARE ELEKTROKARDIOGRAMM (EKG)-VORRICHTUNGEN
APPAREILS D'ÉLECTROCARDIOGRAPHIE (ECG) ÉTIRABLES

(30) Priority: 05.11.2019 US 201962930985 P
(43) Date of publication of application: 21.09.2022
(73) Proprietor: The Board of Trustees of the Leland Stanford Junior University, Stanford, CA 94305-2038 (US)
(72) Inventor: LIU, Yuxin, Redwood City, California 94063 (US); BAO, Zhenan, Stanford, California 94305 (US); KHAN, Yasser, Redwood City, California 94063 (US)
(74) Representative: Hancox, Jonathan Christopher
(86) International application number: PCT/US2020/059124
(87) International publication number: WO 2021/092184

(56) References cited:
- WO-A1-2017/039518
- WO-A1-2017/039518
- WO-A1-2019/165219
- WO-A1-2019/165219
- TW-U- M 579 010
- TW-U- M 579 070
- TW-U- M 579 070
- US-A1- 2007 043 284
- US-A1- 2007 043 284
- US-A1- 2009 062 670
- US-A1- 2009 062 670
- US-A1- 2016 367 163
- US-A1- 2016 367 163
- US-A1- 2019 117 083
- US-A1- 2019 117 083
- US-A1- 2019 223 722
- US-A1- 2019 223 722
- US-B1- 6 230 049
- US-B1- 6 230 049

## Description

### BACKGROUND

Aspects of the present disclosure are directed to a wearable electrocardiogram (ECG) apparatus.

An ECG (electrocardiogram) measures the electrical signal emitted by the heart, which is generated by the propagation of action potentials that cause depolarization of the heart fibers. Physiologically, transmembrane ion currents are generated in the heart during cardiac electrical signals from established traditional chest locations. Cardiac depolarization occurs high in the right atrium within the sinoatrial (SA) node before it extends to the left toward the left atrium and downwards toward the atrioventricular (AV) node. After a delay is caused by the AV node, the depolarization impulse passes through the His bundle and moves with the Purkinje fibers into the right and left bundle branches to activate the right and left ventricles.

During each cardiac cycle, an ionic current generates an electric field in and around the heart, which spans the anterior chest region of the patient's body, the skin to the lower right and lower left of the sternum on the left front chest, and can be detected by ECG electrodes placed on the extremities. Cardiac electrical activity is visually represented in the ECG trace by a PQRSTU waveform, which can be interpreted after ECG recording to derive heart rate and physiology. The P wave represents atrial electrical activity and the QRSTU component represents ventricular electrical activity. Specifically, the P wave represents atrial depolarization, which causes atrial contraction.

P-wave analysis based on ECG monitoring is often an important aspect for accurate cardiac rhythm diagnosis and focuses on identifying the source site and pathway underlying the arrhythmia symptoms. Certain arrhythmias may be difficult to detect clinically. Cardiac rhythm disorders are often sporadic and may not occur in the clinic during a traditional 12-lead ECG. To address the same, traditional multi-lead holter monitor diagnoses require 24-48 hours of wearing, which poses significant inconvenience for users. TW 579 010 U discloses a flexible ECG detection device.

### SUMMARY OF VARIOUS ASPECTS AND EXAMPLES

The invention is defined in independent system claims 1 and 13, and in independent method claim 14. Further aspects and preferred embodiments are defined in the appended claims.

### BRIEF DESCRIPTION OF FIGURES

Various example embodiments, including experimental examples, may be more completely understood in consideration of the following detailed description in connection with the accompanying drawings, each in accordance with the present disclosure, in which:
FIG. 1 is a diagram showing a side, cut-away view of an example apparatus, for illustrating certain exemplary aspects of the present disclosure;
FIG. 2 is another diagram related to FIG. 1, according to certain exemplary aspects of the present disclosure, and showing an example pathway for cardiac-based electrical signals to flow through an apparatus of the type shown in FIG. 1;
FIG. 3 shows an example method of manufacturing a sensor apparatus, in accordance with various example embodiments of the present disclosure;
FIGs. 4A and 4B respectively show two sets of designs which may be used for forming the multi-lead and electrode structures, in accordance with various example embodiments of the present disclosure;
FIGs. 5A and 5B respectively show two sets of designs, also in accordance with various example embodiments of the present disclosure, which may be used for forming material-based structure associated with electrode structures such as those shown in FIGs. 4A and 4B; and
FIGs. 6A, 6B and 6C are related system-direct depictions of an experimentally-developed and/or more-detailed system, in accordance with various example embodiments of the present disclosure, with FIG. 6A showing a patch as may be used in operation on a subject for monitoring/assessing cardiac issues, with FIG. 6B showing an arrangement of circuitry used in the system, and with FIG. 6C showing multi-channel amplification and/or conditioning as may be used to collect and provide electrical signals in connection with FIGs. 6A and 6B.

While various embodiments discussed herein are amenable to modifications and alternative forms, aspects thereof have been shown by way of example in the drawings and will be described in detail. It should be understood, however, that the intention is not to limit the disclosure to the particular embodiments described. On the contrary, the intention is to cover all modifications, equivalents, and alternatives falling within the scope of the disclosure including aspects defined in the claims. In addition, the term "example" as used throughout this application is only by way of illustration, and not limitation.

### DETAILED DESCRIPTION

Aspects of the present disclosure are believed to be applicable to a variety of different types of apparatuses, systems and methods involving a sensor apparatus that may capture a multi-lead electrocardiogram (ECG). While not necessarily so limited, various aspects may be appreciated through a discussion of examples using this context.

Accordingly, in the following description various specific details are set forth to describe specific examples presented herein. It should be apparent to one skilled in the art, however, that one or more other examples and/or variations of these examples may be practiced without all the specific details given below. In other instances, well known features have not been described in detail so as not to obscure the description of the examples herein. For ease of illustration, the same connotation and/or reference numerals may be used in different diagrams to refer to the same elements or additional instances of the same element. Also, although aspects and/or features may in some cases be described in individual figures, it will be appreciated that aspects and/or features from one figure or embodiment can be combined with aspects and/or features of another figure or embodiment even though the combination is not explicitly shown or explicitly described as a combination, and such combinations includes aspects and/features being used together regardless the parts of which the aspects and/or features may be found.

As an example consistent with one specific embodiment of the present disclosure, a multi-layered stretchable sensor apparatus includes a patch having a plurality of stretchable leads, with each of the plurality of stretchable leads including an associated electrode which is to receive electrical signals generated in response to a subject's heart and to pass the received electrical signals along a respective one of plurality of stretchable leads. The patch may integrate the stretchable substrate with the plurality of stretchable leads on one layer facing the subject and with the patch having an area on another other side for circuitry to reside for collecting the electrical signals which are passed along each of the plurality of stretchable leads through an aperture (or via) provided in the stretchable substrate to the circuitry. In various related alternative embodiments, the aperture may be used to define one or more conductive traces for carrying the electrical signals, and may be used to provide dielectric isolation between the conductive traces either with the aperture at least partially filled with dielectric during a manufacturing step, to provide a dielectric-filled aperture in the material, or left open while using air as the dielectric.

In connection with certain other examples, certain aspects of the present disclosure are directed to a multi-lead, small and bandage-like ECG monitoring apparatus that is formed using a stretchable electrode material and that has multiple ECG-monitor leads which can be used to detect a wide range of medical conditions including, for example, detecting and/or recording certain ECG-related events using a wearable light-weight patch-based device with a small form factor (such as a form factor having a square area of, or not exceeding about 3.5" to about 10" (e.g., using a rectangular-shaped patch of dimensions being 1.5" by 3.0", or 2.0" by 3.5") which provides better comfort and improved robustness. For example, when comparing a single-lead ECG to a multi-lead ECG, there can be less than 50% concordance of single-lead ECG recording with a conventional multi-lead cardiac monitor, which is deemed as the "gold standard". In addition, many physicians prefer multi-lead ECG devices compared to single-lead ECG devices, because the single lead ECG can miss some types of arrhythmia such as premature ventricular contractions (PVC).

Using such small dimensions, in more specific examples the plurality of electrodes are spaced apart by distances that are less than a traditional holter ECG (i.e., a portable EKG device that monitors the electrical activity of a person's heart) or standard lead ECG. The processing circuitry can determine the multi-lead ECG by converting the ECG vectors from the plurality of electrodes to ECG vectors associated with the traditional halter ECG or standard lead ECG (such as converting ECG vectors from close-together electrodes to ECG electrodes from far-away or widely spaced electrodes).

Also in accordance with the present disclosure, various other embodiments are directed to an example sensing apparatus which is convenient for a subject (e.g., human patient) to wear and which may result in greater cardiac-directed tracking compliance. As arrhythmia often occurs infrequently, certain examples of the present disclosure are directed to comfortable wearable of a patch for continuous monitoring of the ECG signal to capture transient abnormal cardiac activities. In connection with addressing this issue, certain example embodiments in accordance with the present disclosure provide high signal-sensing accuracy and enhance patient patch-monitoring compliance (associated with device being worn over prolonged periods of time) due to the comfort, for example, as may be attributable to its ability to stretch as manifested by material used to form the patch and related materials integrated with the patch. While other products may be effective tools for screening purposes in general population, many lack high-accuracy capability for adequate arrhythmia diagnosis and may cause discomfort due to tension between the skin and the structure mounted thereon and this tension is understood to affect necessary patient compliance. Moreover, due to size and itchiness and/or the device not remaining on the subject's body, extended and improved compliance is realized by such examples of the present disclosure. As other examples, methods of use based on the above device being worn over prolonged periods of time incudes minimizing surgery due to misdiagnosis ensuing from shorter periods of monitoring, and/or providing and changing medications over the prolonged periods of time while using the worn device for monitoring how the heart is reacting to previous medications.

In various aspects, the sensor apparatus is formed of a stretchable material and has multiple leads. The sensor apparatus couples to a subject and is used to monitor a multi-lead ECG from the subject. In specific aspects, the sensor apparatus can be used to detect a shift in electrical axis, broad QRS complexes, and/or arrhythmias, such as PVC.

The sensor apparatus can include sensor circuitry and processing circuitry. The sensor circuitry includes a plurality of electrodes that can contact a skin surface of a subject, with the plurality of electrodes being interconnected to form the multiple leads. The sensor circuity may include a stretchable substrate having an aperture formed therein, and a first and second conductive and stretchable substrate which overlaps the aperture. The first conductive and stretchable substrate includes the plurality of electrodes arranged in an array and first interconnects that couple the plurality of electrodes to a first end or opening of the aperture. The second conductive and stretchable substrate includes second interconnects coupled to a second end or opening of the aperture. The aperture provides electrical connection between the plurality of electrodes, the first interconnects and the second interconnects. The sensor circuitry can further include an adhesive material coupled to the first conductive and stretchable substrate, and which couples the sensor apparatus to the subject. The adhesive material is positioned to contact other skin surfaces than the plurality of electrodes.

The processing circuitry can include a printed circuit board (PCB) or flexible circuit board (FCB) 160, 260 (which may include flexible IC chips and/or be sufficiently small so as not to require flexible IC chips) coupled to the second interconnects via a bonding contact 150, 250. The processing circuitry obtains electrical signals emitted by the heart of the subject, as captured by the plurality of electrodes, and provides the multi-lead ECG based thereon.

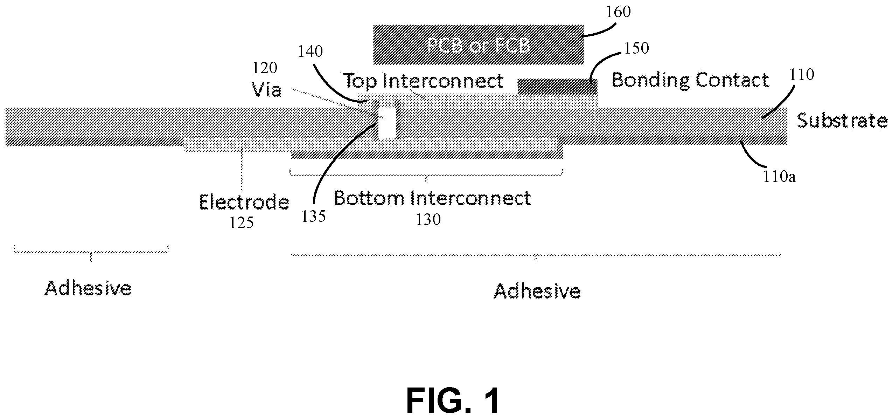

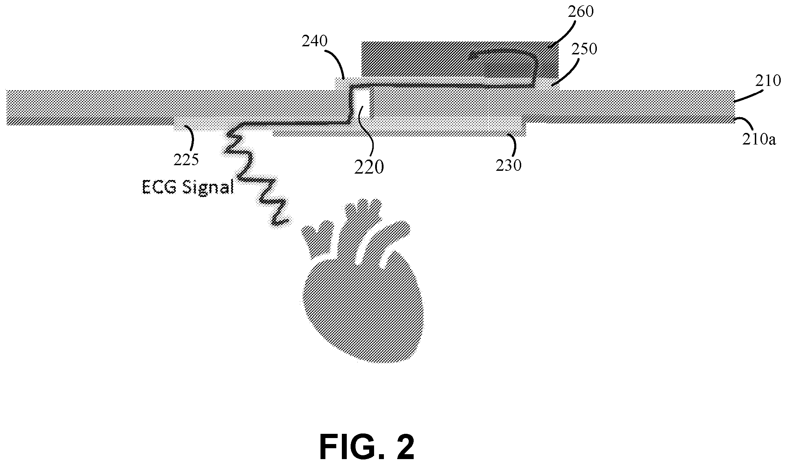

FIG. 1 differs from FIG. 2 in relation to use of the bonding contact 150, 250 and the circuit structure 160, 260. In FIG. 1, the bonding contact 150 is shown below the circuit structure 160 before the circuit structure 160 may be attached to secure to the sensor apparatus or patch, whereas in FIG. 2, the circuit structure 260 and the bonding contact 250 are shown secured to one another in place for use in sensing such electrical signals as discussed above.

Further in accordance with present disclosure, certain aspects are directed to a sensor apparatus including sensor circuitry and processing circuitry as exemplified in connection with the cut-away views of an example sensor apparatus of FIGs. 1 and 2. Structural aspects of FIGs. 1 and 2 are marked with similar reference numerals (e.g., substrate 110, aperture 120, bottom interconnect 130 respectively corresponding to substrate 210, aperture 220, bottom interconnect 230, etc.). In the specific example shown by way of FIGs. 1 and 2, the sensor apparatus includes the substrate 110, 210 as being stretchable and as having an aperture or via 120, 220 formed therein. Optionally, the stretchable substrate 110, 210 may be characterized as having first and second conductive and stretchable substrate portions with the first portion being at the core and providing sufficient structure for integrating the various materials and circuitry used with the apparatus, and with the second substrate portion 110a, 210a beneath the core and providing adhesion and increasing strength for the patient- or subject-facing side of the patch or sensing apparatus.

Each such substrate portion may be understood with reference to implementation via one or more layers of the overall material-based apparatus integrated with the substrate 110, 210. On the bottom (e.g., patient-facing side), the first conductive and stretchable portion may include a plurality of electrodes arranged in an array and associated first interconnects that couple the plurality of electrodes to conductive traces such as a representative conductive trace 135 (FIG. 1) in an aperture 120, 220. In various more-specific examples, one or more larger apertures may be used to accommodate multiple conduction paths associated with the respective electrodes, each with multiple conductive traces (e.g., as part of the leads) connecting to respective multiple electrodes. In various examples, a single aperture have multiple conductive traces for leads associated with each of 2, 3, 4 or 6 electrodes, in another example, two apertures may be used so that each aperture accommodates 3 conductive traces for leads associated with a total of 6 electrodes, etc. More generally, in one example, N apertures may be used so that each aperture accommodates N (or alternatively more than N) conductive traces for leads associated with a total of N (or alternatively more than N) electrodes facing the patient, where N is an integer greater than or equal to 1 (and practicably, not greater than about 11).

One such electrode is depicted as 125, 225, and one such interconnect for the electrode is depicted as 130, 230. Using the interconnect 130, for example, to the right side of FIG. 1 a corresponding lead 130a interconnect130, The plurality of electrodes may contact a subject and/or capture electrical signals emitted by the subject's heart at the chest area (or other nerve region of the subject). On the opposite or top side opposing the patient-facing side, the second conductive and stretchable substrate portion includes second interconnects such as interconnect 140, 240, that couple to corresponding conductive traces such as the conductive trace 135 (e.g., FIG. 1) in the aperture 120, 220 from the top side.

The aperture may provide a pathway for the electrical connection between one or more of the plurality of electrodes (e.g., 125 of FIG. 1) to an upper side of the substrate whereat a circuit area is located as over the interconnect 140 (e.g., FIG. 1). An adhesive material is implemented as the bonding contact 150, 250 in FIGs. 1 and 2, on top of the interconnect 140, 240. Sensor circuitry (such as including at least analog front end circuitry for conditioning and/or amplifying the electrical signals) may be implemented using a circuit structure or circuit board 160, 260 which may be coupled to the interconnect 140, 240 via the bonding contact 150, 250 so that its adhesive material couples or attaches the sensor apparatus to the subject. The circuit structure or circuit board may be, for example, a printed and/or flexible circuit board sometimes known as PCB or FCB The processor circuitry obtains the electrical signals as captured by the plurality of electrodes and provide a multi-lead ECG signal based on the electrical signals.

In a number of embodiments, processing circuitry and/or additional logic circuit is configured and arranged to respond to the electrical signals by tracking and scoring a set of ECG vectors with weighted factors and/or based on or using artificial intelligence (AI) empirical models to analyze the set of ECG vectors relative to a larger set of empirically-obtained cardiac data. Such processing circuitry may be part of the PCB or FCB based circuitry as above and/or other remotely-situated circuitry such as a CPU or smartphone programmed to receive and process such signals as may be conventional. The remotely-situated circuitry may be wirelessly coupled (e.g., via Bluetooth or infrared) or coupled via a wired connection.

In various more-detailed/experimental examples, different materials may be chosen to provide such an integrated structure. Using representative FIG. 1, the following are non-limiting examples. The substrate 110 may be formed using a stretchable polymer (e.g., Proflex SEBS, Polyurethane or PMMA based polymer (Kurray LA series)), and the second substrate portion 110a may be formed using a Stretchable Adhesive Polymer material-based layer (e.g., a mixture of MG7-9850 (Providing Adhesion) and/or PDMS for increasing strength (in one example, Dragon Skin 0010 was found useful for the second substrate portion 110a). The traces 135 along the sidewall of the aperture 120, as well as the top interconnect 140 and the electrode 125, may be formed using one or more of: a stretchable polymer which is conductive or a stretchable mixture of materials including a polymer with sufficient conductive material so as not to noticeably impede the low-level electrical signals as such those being collected from the heart and for ECG analysis. The bonding contact 150 may be formed using one or more anisotropic conductive materials such as commercially-available adhesive tape (e.g., ACF Tape).

The sensor circuitry in the circuit structure may have low-impedance electrically-conductive hydrogel (ECH) electrodes and implemented using a flexible ultrathin printed circuit board (PCB). For example, such a PCB may be designed to communicate with other devices, such as a smartphone through Bluetooth so that a subject (e.g., a patient) can easily store captured data locally or share with a physician. Enabled by a versatile fabrication process for an ECH electrode array, multi-lead electrodes can be patterned at customized locations. Such an ECH electrode is a dual conductor with both ionic and electrical conductivity, and therefore the single material can replace two component metal/gel electrodes that are currently used for conventional ECG electrode and provide lower impedance at the same time. The low impedance of ECH materials allow for miniaturizing of the ECG electrodes and while still providing good signal-to-noise ratio. Multi-lead ECG traces (e.g., 2-9) can be recorded with low power analog front end.

In use, with the sensor apparatus formed of stretchable material and with multiple leads, the sensor apparatus couples to a subject using adhesive (bottom of FIG. 1) and is configured to monitor a multi-lead ECG from the subject. In specific example embodiments, the sensor apparatus cam detect a shift in electrical axis, broad QRS complexes, and/or arrhythmias, such as PVC. In other related specific examples, the plurality of patient-engaging electrodes are spaced apart by distances that are less than a traditional holter ECG or standard lead ECG. The processing circuitry can determine the multi-lead ECG by converting the ECG vectors from the plurality of electrodes to ECG vectors associated with the traditional halter ECG or standard lead ECG (such as converting ECG vectors from close-together electrodes to ECG electrodes from far-away or widely spaced electrodes). The conversion may be based on or using machine learning functions and/or artificial intelligence (AI) models to analyze sets of ECG vectors relative to a larger set of empirically-obtained cardiac data.

Such processor circuitry (as in the illustrated PCB or FCB) may be configured to obtain the electrical signals as captured by each of the electrodes and provide a multi-lead ECG signal based on the electrical signals. Each lead represents differences in electrical potentials measured in two (or more) points in space, and can be represented as a graph or vector which impacts the ECG curve or signal.

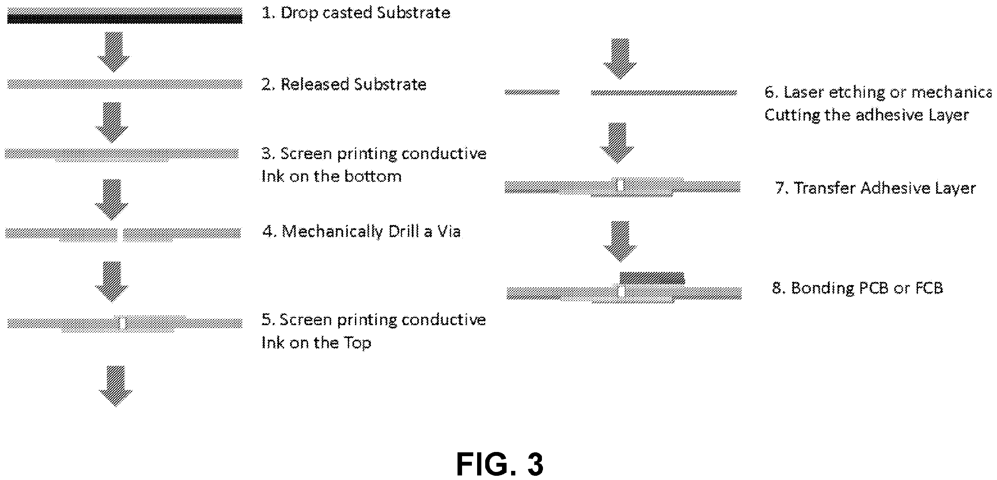

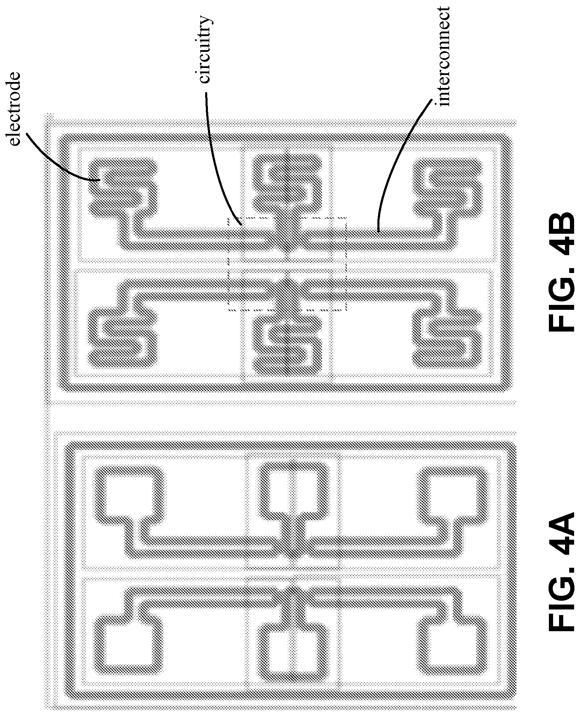

FIG. 3 shows an example method of manufacturing a sensor apparatus, in accordance with various embodiments such as described in connection with FIGs. 1 and 2 and/or other embodiments such as the following example embodiment involving another patch or skin-wearable sensor apparatus also made using stretchable materials. Accordingly, the following example embodiment includes a stretchable substrate having at least one aperture formed therein as described above (e.g., provided after molding by drilling or during molding by use of a post to occupy the aperture area). Further, the example embodiment includes a first conductive and stretchable substrate (e.g., having a bottom side electrode), and a second conductive and stretchable substrate (e.g., having a top side interconnect), and the first conductive and stretchable substrate includes a plurality of electrodes arranged in an array through which the plurality of electrodes are arranged for leading electrical signals to the aperture(s). The electrodes can contact a subject and/or otherwise be coupled adjacent the subject's skin in a sufficiently close manner so as capture electrical signals emitted by the subject's heart. Adhesive material is used to couple and/or attach the sensor apparatus to the subject, similar to a band-aid and, depending on the implementation, the electrodes may or may not be covered by the adhesive material. In the above examples and other related embodiments, adhesive material (for example, tape or film) maybe applied or attached to areas adjacent or orthogonal to the electrodes. In this manner, one set of adhesive material may cover dielectric regions and another set of adhesive material may cover the regions for the electrodes. This may be appreciated with respect to the views shown in FIGs. 4A-5B.

An exemplary set of steps for forming the flexible core portions of such example embodiments are shown in FIG. 3. In step 1 of FIG. 3, the stretchable substrate is drop casted, and at step 2 the substrate is released. In a specific experimental embodiment, Proflex SEBS or SEBS 1062 may be dissolved in 20% toluene and the solution may be drop casted on glass slides (e.g., 3 inch x 2 inch slides). In this manner, the SEBS substrate layer may be readily released from the glass slides. In step 3, conductive ink is formed (e.g., screen printed) on a first side of the stretchable substrate to form the first conductive and stretchable substrate. In a specific experimental embodiment, a mechanical cutter can be used to cut a mask (made of PET) for the bottom interconnects and the stretchable conductor (e.g., Dupont PE874) can be screen printed to form the bottom interconnect. The substrate can be heated (e.g., back heated) at 110 degrees C for 10min on a hot plate. In step 4, an aperture (e.g., the via) may be formed, such as by mechanically drilling a via. For example, a mechanical punch with diameter of 3mm can be used to cut a circular hole or via on the stretchable substrate. As noted previously, however, the aperture may be provided as part of step 1 with a post. In a specific embodiments, the punch/hole can penetrate both the SEBS substrate and the printed stretchable conductor (e.g., both the stretchable substrate and the first conductive and stretchable substrate).

In step 5, conductive ink is formed (e.g., screen printed) on a second side of the stretchable substrate to form the second conductive and stretchable substrate. The stretchable substrate is flipped and a mechanical cutter is used to cut a mask (made of PET) for the top interconnects (e.g., the second conductive and stretchable substrate). The conductive and stretchable substrate can be formed by screen printing stretchable conductor (e.g. Dupont PE874) to form the top interconnect. The substrate is heated (e.g., baked), such as at 110°C for 10min on a hot plate.

At step 6, the adhesive material is laser etched or mechanically cut. In a specific experimental embodiment, this can involve oxygen plasma treatment of a separate substrate (e.g. 2x3 inch glass slides) for 45 seconds at 300Watts, and 150 torr and spin coating Dextran (5% in water) on the glass to form a sacrificial layer. An adhesive polymer (e.g. MG7-9850) can be mixed with PDMS with the following example ratio: MG7-9850-PartA: MG7-9850-Part-B: DragonSkin-PartA: DragonSkin-PartB =1:1:0.02:0.02 (weight ratio); and use speed mix to mix the compounds at 3000 rpm for 5 minutes and the mixed compound may be spin coated on the dextran coated glass slides at 2000 rpm for 1min and dried overnight or cured at 70°C for 1 hour. The glass/dextran/adhesive can be bonded to the substrate from step 5. After bonding, the substrate and bonded glass/dextran/adhesive can be placed into a DI water bath to release the adhesive from the glass (within 1 hour).

In step 7, the adhesive layer is transferred to the sensor circuitry, such as to portions of the stretchable substrate and the first conductive and stretchable substrate. For example, anisotropic conductive tape can be used to paste on the contact on the top interconnect, and the contact may be used to attach to a FCB or PCB. Step 8 shows such a PCB or FCB bonded to the sensor circuitry proximal to the second conductive and stretchable substrate.

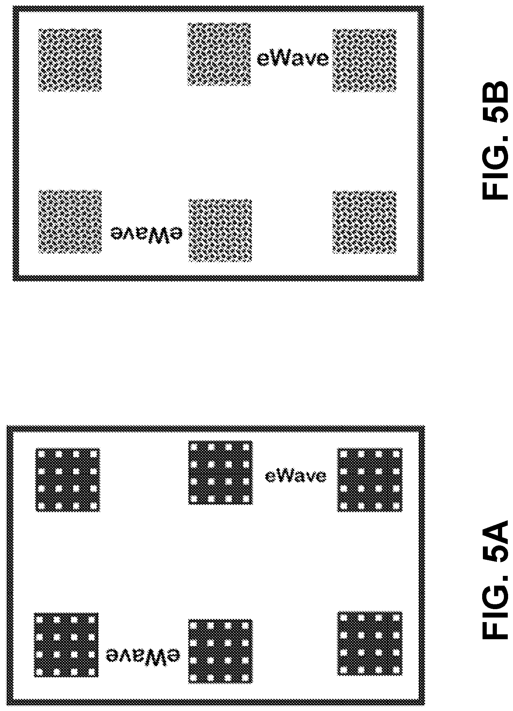

FIGs. 4A-4B and 5A-5B illustrate examples of masks that may be used on connection with manufacturing the electrodes, such as described in the above exemplary method of FIG. 3. In connection with step 3 of FIG. 3, FIGs. 4A and 4B respectively show a pair of alternative potential (e.g., PET) masks that may be used for forming a first conductive and stretchable substrate in which an array is provided having six electrodes and associated leads or interconnects (shown along a parallel pair of elongated outlines connecting to the six electrodes). The above-noted circuitry area may be located over/below one or more locations around the array (e.g., in the middle of the illustrations of FIGs. 4A and 4B. In connection with step 5 of FIG. 3, FIGs. 5A-5B illustrate different alternative (e.g., PET) masks as may be used for forming a second conductive and stretchable substrate with screen printing for the six patient-engaging electrodes. While six electrodes are used in such illustrations, according to certain other examples consistent with the present disclosure, a greater or a fewer number of electrodes may be used and such patterned electrodes may also be used in combination with one or more additional electrodes/leads not limited to the confines of the patch or sensing apparatus. In certain examples, instead of six as illustrated by FIGs. 4A-5B, three to seven or nine electrodes may be used. Additionally, the placement of the electrodes, distance between the electrodes, and/or the number of electrodes can be adjusted based on a type of arrhythmia condition being monitored and/or detected.

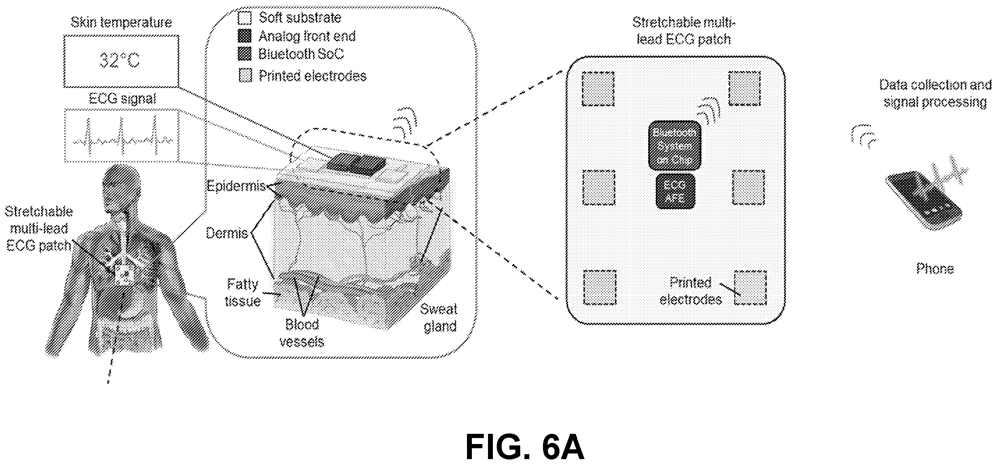

FIGs. 6A-6C illustrate an example of a sensor apparatus as attached to a skin surface of a subject as part of a collection and analysis system to depict an experimentally-developed and/or more-detailed example, also in accordance with the present disclosure. In FIG. 6A, a sensor apparatus or patch is attached to the chest of a patient via the adhesive material and is used to monitor a multi-lead ECG from electrical signals emitted from the heart of the patient and as captured by the electrodes of the sensor apparatus. The sensor apparatus or patch of FIG. 6A is shown with six electrodes for engaging related areas of the chest and with patch-mounted circuitry as including: a thermistor or other temperature-sensing circuit for tracking the temperatures of the sensor apparatus and/or the chest area of the patient, an EDG analog front end (AFE) circuit for conditioning (e.g., filtering noise and/or signal amplification), and a Bluetooth system on a chip to wirelessly transmit the conditioned electrical signals to an external CPU or PDA (personal device assistant) such as a smartphone.

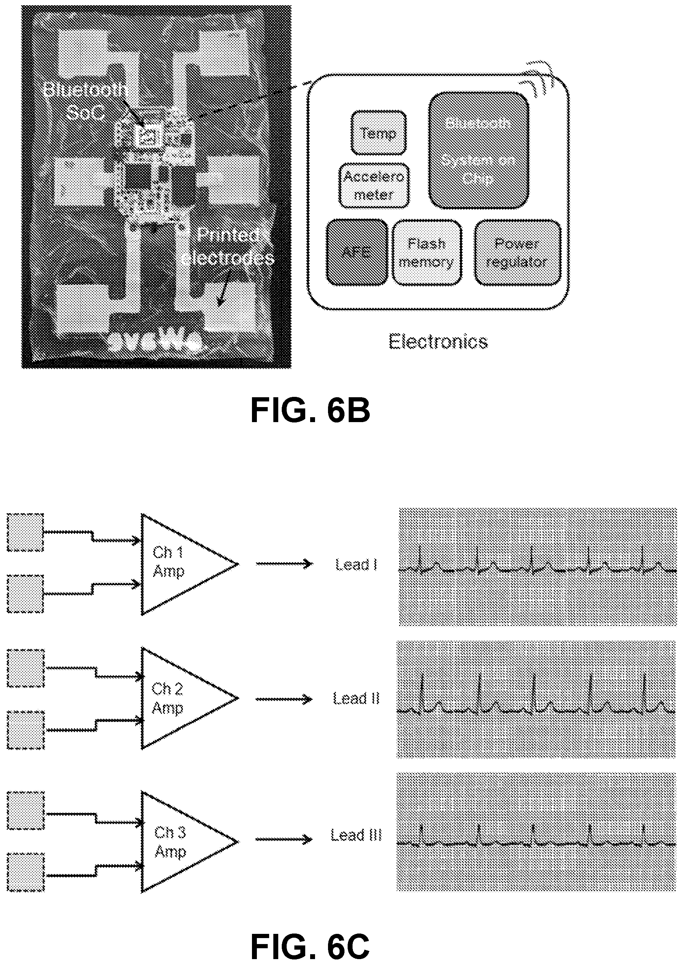

FIG. 6B shows an arrangement of such circuitry used in the patch, and with FIG. 6C showing multi-channel amplification/conditioning circuitry (e.g., a plurality of dual-input buffer-type amplifiers) as may be used to collect and provide electrical signals in connection with FIGs. 6A and 6B. The multi-channel amplification/conditioning circuitry may be associated with a selection input, as may be provided by a controller (e.g., part of the Bluetooth system on a chip), to select signals from one as opposed to both inputs and, alternatively or in combination, the multi-channel amplification/conditioning circuitry may have a buffer-amplifier with its dual inputs for conditioning and/or amplifying the electrical signals for one of three ECG leads (lead I, lead II and lead III), whereby three such buffer-amplifiers as illustrated are configured for concurrently use so as to condition and/or amplifying electrical signals respectively for each of the three ECG lead I, lead II and lead III with each such lead being associated with a different ECG-related signal as shown on the far right of FIG. 6C.

In certain experimental example embodiments, exemplary data is obtained. More specifically, using such a system as in FIGs. 6A, 6B and 6C, resulting ECG signals monitored by a sensor apparatus in accordance with various embodiments as described above (e.g., in connection with FIGs. 1A and 1B and other such examples), the ECG signals are obtained when the patient is walking and sitting, and used to output a classified sinus rhythm by the processing circuitry.

Using certain of the above-characterized aspects of the present disclosure, certain example implementations may be used to realize significant advantages. Such advantages include, for example, stretchable electronic technology to provide a nearly imperceptible bandage-like electronic sensor that continuously monitor for multi-lead ECG. The feasibility of making such a bandage-like apparatus has been established experimentally via example prototypes having a weight in the range of 3 grams to 7 grams (for example 5 grams on average) and small size (such as having width of 5 cm, length of 8 cm and thickness of 0.2 cm), thereby providing evidence that such accurately-sensing flexible apparatus may be implemented with at least 10x lower thickness and 6x reduction in weight compared with known continuous-arrhythmia detection solutions. Moreover, in certain examples for providing improved comfort and more stable signal recording of ECG, all of the materials in the stretchable band-aid may be composed of stretchable material. Further, while portable or wearable single-lead ECG patches are known, important information is missed from having additional (other) ECG leads as may be used for both automatic algorithm analysis and physician interpretation. For example, compared to single-lead, multi-lead recordings allow for the detection of shift in electrical axis and improved detection of aberrant/broader QRS complexes and detection of certain types of arrhythmias such as premature ventricular contraction (PVC).

Additional/related benefits of using such two-input amplifiers, in combination with electrodes situated in such a small area as defined by the example patch-like embodiments of the present disclosure, may be appreciated relative to the use conventionally ECG electrodes being placed far apart from each other on a chest of a patient. For example, four standard Limb electrodes, including RL, LL, RA, LA are often to be used for standard lead I, II and II measurements while the electrodes are separated with two at each shoulder and the other two at the upper thigh. The ECG vector can be measured by the potential difference across each pair of electrodes. Conventional Standard Lead I, II, and III may be respectively represented by a triangular association of the electrode arrangement with an electrode pair associated with the two shoulders, another pair associated with the right shoulder and the left thigh, and a third pair (third leg of the triangle) associated with the left shoulder and the left thigh. Using one of the example embodiments as above and according to the present disclosure, an ECG patch is realized with electrode array and with the ECG vector being measured from a reduced spacing between the electrodes on the ECG patch. The (close-proximity) vectors measured on the patch may be referred to as Lead-I-close, Lead-II-close, and Lead-III-close as they may correspond directly to the orientation involving the triangular association as described above but using a fraction of the displacement distances. An electrode in an unused corner of the patch may be used as the reference electrode.

Using such examples as in connection with the ECG patch providing the close-proximity vectors, methods of using a sensing apparatus (e.g., as illustrated in FIGs. 1- 2 of the present disclosure) employ use of a logic circuit (e.g., a CPU) configured to respond to electrical signals (processed as vectors) received from each of the electrodes by tracking and scoring each vector with a weighting factor (each assigned weight can be based on a likelihood that a detected vector is sufficiently strong in terms of signal strength and/or likelihood of match to an expected vector found in empirical data) and then analyzing sets of vectors relative to a larger set of empirically-obtained cardia data (e.g., such data collected from sample patients with certain heart conditions/health heart similar modeled in 2 and/or 2+ dimensional tables by previous clinical testing). In related and more-specific embodiments, this above approach is used with a hierarchical organization of such empirical data (e.g., organized based on factors indicating correlation with clinicals having heart-specific physiologic similarities/demographics). Based on the analyses of the sets of such vectors, further collection and analysis is adjusted (as part of the AI) so as to focus on vectors developed from fewer than all of the electrodes which are part of the apparatus. From these contexts and/or AI methodologies, the patch or sensing apparatus may be used to assess and diagnose cardiac conditions as may be known or reported in the literature. For example, it has been reported that the direction of the vector (revealing abnormal heart axis) can change under different circumstances such as follows: (i) When the heart itself is rotated (right ventricular overload), the axis turns with it. (ii) In case of ventricular hypertrophy, the axis can deviate toward the greater electrical activity and the vector cam turn toward the hypertrophied tissue. (iii) Infarcted tissue is electrically dead. No electrical activity is registered and the QRS vector turns away from the infarcted tissue. (iv) In conduction problems, the axis can additionally deviate. When the right ventricle depolarizes later than the left ventricle, the axis will turn to the right (RBBB). This is because the right ventricle can begin the contraction later and therefore cam also finish later. In a normal situation, the vector is influenced by the left ventricle, but in RBBB only the right ventricle determines it.

Accordingly, the above provides a sample of example conditions that can be assessed from such methodology in accordance with certain example embodiments of the instant disclosure. Embodiments are not limited to the above-listed circumstances, and other exemplary embodiments are contemplated.

It is recognized and appreciated that as specific examples, the above-characterized figures and discussion are provided to help illustrate certain aspects (and advantages in some instances) which may be used in the manufacture of such structures and devices. These structures and devices include the exemplary structures and devices described in connection with each of the figures as well as other devices, as each such described embodiment has one or more related aspects which may be modified and/or combined with the other such devices and examples as described hereinabove may also be found in the Appendices of the above-referenced Provisionals.

The skilled artisan would also recognize various terminology as used in the present disclosure by way of their plain meaning. As examples, the Specification may describe and/or illustrates aspects useful for implementing the examples by way of various semiconductor materials/circuits which may be illustrated as or using terms such as layers, blocks, modules, device, system, unit, controller, and/or other circuit-type depictions. Such semiconductor and/or semiconductive materials (including portions of semiconductor structure) and circuit elements and/or related circuitry may be used together with other elements to exemplify how certain examples may be carried out in the form or structures, steps, functions, operations, activities, etc. It would also be appreciated that terms to exemplify orientation, such as upper/lower, left/right, top/bottom and above/below, may be used herein to refer to relative positions of elements as shown in the figures. It should be understood that the terminology is used for notational convenience only and that in actual use the disclosed structures may be oriented different from the orientation shown in the figures. Thus, the terms should not be construed in a limiting manner.

Based upon the above discussion and illustrations, those skilled in the art will readily recognize that various modifications and changes may be made to the various embodiments without strictly following the exemplary embodiments and applications illustrated and described herein. For example, methods as exemplified in the Figures may involve steps carried out in various orders, with one or more aspects of the embodiments herein retained, or may involve fewer or more steps.

## Claims

1. An apparatus comprising:
a plurality of stretchable leads, each of the plurality of stretchable leads including a stretchable polymer and an associated electrode which is to receive electrical signals generated in response to a subject's heart and to pass the received electrical signals along a respective one of plurality of stretchable leads; and
a patch including a stretchable substrate (110, 210) integrated with the plurality of stretchable leads and including a circuitry area for circuitry (160, 260) to reside for collecting the electrical signals passed along each of the plurality of stretchable leads, wherein each of the plurality of stretchable leads is to be on a subject side of the patch.

2. The apparatus of claim 1, further including the circuitry (160, 260), and wherein the circuitry is to be secured at the circuitry area of the patch and to be coupled the plurality of stretchable leads, and wherein the stretchable polymer has elastic properties.

3. The apparatus of claim 1, wherein the stretchable substrate (110, 210) has an at least one aperture (120, 220) formed therein and further has a conductor overlapping said at least one aperture, wherein the conductor overlapping said at least one aperture is to pass the received electrical signals from the plurality of stretchable leads along a path leading to the circuitry area.

4. The apparatus of claim 1, further including the circuitry (160, 260) secured at the circuitry area, and wherein the circuitry includes a controller having configurable or programmable logic circuitry and further includes: a plurality of multi-channel amplifiers, wherein each of the multi-channel amplifiers is configured according to at least one of the following: being capable of selectively amplifying one of the electrical signals in response to selectivity control provided by the controller, and having a pair of inputs as an ECG lead.

5. The apparatus of claim 1, wherein the plurality of electrodes includes four to nine individual electrodes for placement against the subject via the patch, and wherein the patch is to occupy less than seven square inches of skin area of the subject while worn on the subject.

6. The apparatus of claim 1, wherein the circuitry (160, 260) is to condition and amplify the electrical signals to sense one or more of: a shift in electrical axis, broad QRS complexes, and a cardiac arrhythmia.

7. The apparatus of claim 1, wherein the patch includes a first material layer having the stretchable substrate (110, 210), a second material layer (110a, 210a) on one side of the first material layer and having the plurality of stretchable leads, and at least one aperture (120, 220) formed through the first material layer with a conductor overlapping said at least one aperture, wherein an interconnect (140, 240) is to be located on another opposing side of the first material layer and to be secured to the circuitry (160, 260) at the circuitry area.

8. The apparatus of claim 7, wherein said at least one aperture (120, 220) is to act as a dielectric and a pathway for the conductor to carry the electrical signals from the one side to the other side of the first material layer.

9. The apparatus of claim 7, wherein the substrate (110, 210) is formed via dropcast as a solid film, the plurality of electrodes and the plurality of stretchable leads are screen-printed conductive ink, and the circuitry includes screen printed conductive ink as traces for the circuitry on the other opposing side of the first material layer.

10. The apparatus of claim 1, further including:
the circuitry (160, 260), and wherein the circuitry is to be secured at the circuitry area of the patch and to be coupled the plurality of stretchable leads; and
a data collection and signal processing circuit, including a receiver circuit to receive conditioned signals derived from the electrical signals and including a memory circuit for storing the conditioned signals.

11. The apparatus of claim 10, wherein the data collection and signal processing circuit includes or is part of computer circuit implemented with a user interface and configured to provide information associated with an ECG.

12. The apparatus of claim 1, where conductive adhesives are attached to the electrodes, while insulating adhesives are attached to areas orthogonal to the electrodes on the substrate.

13. An apparatus comprising:
sensor circuitry including:
a stretchable substrate having an aperture (120, 220) formed therein;
a first conductive and stretchable substrate (110, 210) including a plurality of electrodes arranged in an array and first interconnects (140, 240) that couple the plurality of electrodes to a first opening of the aperture, the plurality of electrodes being configured and arranged to contact a subject and/or to capture electrical signals emitted by a heart of the subject;
a second conductive and stretchable substrate (110a, 210a) including second interconnects (130, 230) that couple to a second opening of the aperture, the aperture to provide electrical connection between the plurality of electrodes, the first interconnects, and the second interconnects; and
an adhesive material coupled to the first conductive and stretchable substrate, the adhesive material configured and arranged to couple the sensor apparatus to the subject; and
processor circuitry configured and arranged with the sensor circuitry to obtain the electrical signals as captured by the plurality of electrodes and to provide a multi-lead ECG based on the electrical signals.

14. A method comprising:
receiving electrical signals generated in response to a subject's heart by using electrodes respectively associated with a plurality of stretchable leads, each including a stretchable polymer, as part of a patch having a stretchable substrate (110, 210), and wherein the received electrical signals are passed along a respective one of plurality of stretchable leads while each of the plurality of stretchable leads faces a subject side of the patch, wherein the patch includes a stretchable substrate integrated with the plurality of stretchable leads and including a circuitry area for circuitry to reside; and
using the circuitry to collect the electrical signals passed along each of the plurality of stretchable leads.

15. The method of claim 14, further including using the circuitry to amplify different ones of the electrical signals in response to a configurable and/or programmable user setting, wherein the stretchable polymer has elastic properties.

16. The apparatus of claim 13, including a controller having configurable or programmable logic circuitry and further including: a plurality of multi-channel amplifiers, wherein each of the multi-channel amplifiers is configured according to at least one of the following: being capable of selectively amplifying one of the electrical signals in response to selectivity control provided by the controller, and having a pair of inputs configured as an ECG lead.

17. The apparatus of claim 13, further including a patch associated with the plurality of electrodes, wherein the plurality of electrodes includes from four to nine individual electrodes for placement against the subject and wherein the patch is to occupy less than seven square inches of skin area of the subject while worn on the subject.

18. The apparatus of claim 13, further including circuitry (160, 260) to condition and amplify the electrical signals to sense one or more of: a shift in electrical axis, broad QRS complexes, and a cardiac arrhythmia.

19. The apparatus of claim 13, wherein the plurality of electrodes are associated with a plurality of stretchable leads and a patch that includes: a first material layer having the stretchable substrate (110, 210), a second material layer (110a, 210a) on one side of the first material layer and having the plurality of stretchable leads, and at least one aperture (120, 220) formed through the first material layer with a conductor overlapping said at least one aperture, wherein an interconnect (140, 240) is to be located on another opposing side of the first material layer and to be secured to at least one of the sensor circuitry and the processor circuitry wherein said at least one aperture is to act as a dielectric and a pathway for the conductor to carry the electrical signals from the one side to the other side of the first material layer.

## Patentansprüche

1. Vorrichtung, umfassend:
eine Vielzahl von dehnbaren Leitungen, wobei jede aus der Vielzahl von dehnbaren Leitungen ein dehnbares Polymer und eine assoziierte Elektrode beinhaltet, die elektrische Signale empfangen soll, die als Reaktion auf das Herz eines Subjekts erzeugt werden, und die empfangenen elektrischen Signale entlang einer jeweiligen aus der Vielzahl von dehnbaren Leitungen geben soll; und
ein Pflaster, das ein dehnbares Substrat (110, 210) beinhaltet, das in die Vielzahl von dehnbaren Leitungen integriert ist und einen Schaltungsbereich für Schaltung (160, 260) beinhaltet, um sich zum Sammeln der elektrischen Signale, die entlang jeder aus der Vielzahl von dehnbaren Leitungen gegeben werden, aufzuhalten, wobei jede aus der Vielzahl von dehnbaren Leitungen auf einer Subjektseite des Pflasters sein soll.

2. Vorrichtung nach Anspruch 1, ferner beinhaltend die Schaltung (160, 260) und wobei die Schaltung an dem Schaltungsbereich des Pflasters zu sichern und an die Vielzahl von dehnbaren Leitungen zu koppeln ist, und wobei das dehnbare Polymer elastische Eigenschaften aufweist.

3. Vorrichtung nach Anspruch 1, wobei das dehnbare Substrat (110, 210) zumindest eine Ausnehmung (120, 220) aufweist, die darin gebildet ist, und ferner einen Leiter aufweist, der die zumindest eine Ausnehmung überlappt, wobei der Leiter, der die zumindest eine Ausnehmung überlappt, die empfangenen elektrischen Signale von der Vielzahl von dehnbaren Leitungen entlang eines Pfads, der zu dem Schaltungsbereich führt, geben soll.

4. Vorrichtung nach Anspruch 1, ferner beinhaltend die Schaltung (160, 260), die an dem Schaltungsbereich gesichert ist, und wobei die Schaltung eine Steuerung beinhaltet, die konfigurierbare oder programmierbare Logikschaltung aufweist, und ferner Folgendes beinhaltet: eine Vielzahl von Mehrkanalverstärkern, wobei jeder der Mehrkanalverstärker gemäß zumindest einem des Folgenden konfiguriert ist: in der Lage zu sein, selektiv eines der elektrischen Signale als Reaktion auf Selektivitätssteuerung, die durch die Steuerung bereitgestellt ist, zu verstärken, und ein Paar von Eingängen als eine EKG-Leitung aufweisend.

5. Vorrichtung nach Anspruch 1, wobei die Vielzahl von Elektroden vier bis neun einzelne Elektroden zur Platzierung gegen das Subjekt über das Pflaster beinhaltet und wobei das Pflaster weniger als sieben Quadratzoll von Hautbereich des Subjekts einnehmen soll, während es an dem Subjekt getragen wird.

6. Vorrichtung nach Anspruch 1, wobei die Schaltung (160, 260) die elektrischen Signale konditionieren und verstärken soll, um eines oder mehrere von Folgendem zu erfassen: einer Verschiebung in elektrischer Achse, breiten QRS-Komplexen und einer Herzarrhythmie.

7. Vorrichtung nach Anspruch 1, wobei das Pflaster eine erste Materialschicht, die das dehnbare Substrat (110, 210) aufweist, eine zweite Materialschicht (110a, 210a) auf einer Seite der ersten Materialschicht und die Vielzahl von dehnbaren Leitungen aufweisend, und zumindest eine Ausnehmung (120, 220), die durch die erste Materialschicht gebildet ist, mit einem Leiter, der die zumindest eine Ausnehmung überlappt, beinhaltet, wobei sich eine Zwischenverbindung (140, 240) auf einer anderen gegenüberliegenden Seite der ersten Materialschicht befinden soll und an der Schaltung (160, 260) an dem Schaltungsbereich zu sichern ist.

8. Vorrichtung nach Anspruch 7, wobei die zumindest eine Ausnehmung (120, 220) als ein Dielektrikum und ein Pfadweg für den Leiter dienen soll, um die elektrischen Signale von der einen Seite zu der anderen Seite der ersten Materialschicht zu transportieren.

9. Vorrichtung nach Anspruch 7, wobei das Substrat (110, 210) über Dropcast als ein fester Film gebildet ist, die Vielzahl von Elektroden und die Vielzahl von dehnbaren Leitungen leitende Siebdrucktinte sind und die Schaltung leitende Siebdrucktinte als Spuren für die Schaltung auf der anderen gegenüberliegenden Seite der ersten Materialschicht beinhaltet.

10. Vorrichtung nach Anspruch 1, ferner beinhaltend:
die Schaltung (160, 260) und wobei die Schaltung an dem Schaltungsbereich des Pflasters zu sichern und an die Vielzahl von dehnbaren Leitungen zu koppeln ist; und
eine Datensammlungs- und Signalverarbeitungsschaltung, beinhaltend eine Empfängerschaltung, um konditionierte Signale zu empfangen, die von den elektrischen Signalen stammen, und beinhaltend eine Speicherschaltung zum Speichern der konditionierten Signale.

11. Vorrichtung nach Anspruch 10, wobei die Datensammlungs- und Signalverarbeitungsschaltung Computerschaltung beinhaltet oder Teil davon ist, die mit einer Benutzerschnittstelle implementiert und konfiguriert ist, um Informationen bereitzustellen, die mit einem EKG assoziiert sind.

12. Vorrichtung nach Anspruch 1, wobei leitende Klebstoffe an den Elektroden angebracht sind, während isolierende Klebstoffe an Bereichen orthogonal zu den Elektroden auf dem Substrat angebracht sind.

13. Vorrichtung, umfassend:
Sensorschaltung, beinhaltend:
ein dehnbares Substrat, das eine Ausnehmung (120, 220) aufweist, die darin gebildet ist;
ein erstes leitendes und dehnbares Substrat (110, 210), das eine Vielzahl von Elektroden, die in einem Array angeordnet ist, und erste Zwischenverbindungen (140, 240), welche die Vielzahl von Elektroden an eine erste Öffnung der Ausnehmung koppeln, beinhaltet, wobei die Vielzahl von Elektroden konfiguriert und angeordnet ist, um ein Subjekt zu kontaktieren und/oder um elektrische Signale einzufangen, die durch ein Herz des Subjekts emittiert werden;
ein zweites leitendes und dehnbares Substrat (110a, 210a), das zweite Zwischenverbindungen (130, 230) beinhaltet, die an eine zweite Öffnung der Ausnehmung koppeln, wobei die Ausnehmung elektrische Verbindung zwischen der Vielzahl von Elektroden, den ersten Zwischenverbindungen und den zweiten Zwischenverbindungen bereitstellen soll; und
ein Klebstoffmaterial, das an das erste leitende und dehnbare Substrat gekoppelt ist, wobei das Klebstoffmaterial konfiguriert und angeordnet ist, um die Sensorvorrichtung an das Subjekt zu koppeln; und
Prozessorschaltung, die mit der Sensorschaltung konfiguriert und angeordnet ist, um die elektrischen Signale wie durch die Vielzahl von Elektroden eingefangen zu erhalten und um ein EKG mit mehreren Leitungen basierend auf den elektrischen Signalen bereitzustellen.

14. Verfahren, umfassend:
Empfangen von elektrischen Signalen, die als Reaktion auf das Herz eines Subjekts erzeugt werden, durch Verwenden von Elektroden, die jeweils mit einer Vielzahl von dehnbaren Leitungen assoziiert sind, die jeweils ein dehnbares Polymer beinhalten, als Teil eines Pflasters, das ein dehnbares Substrat (110, 210) aufweist, und wobei die empfangenen elektrischen Signale entlang einer jeweiligen aus der Vielzahl von dehnbaren Leitungen gegeben werden, während jede aus der Vielzahl von dehnbaren Leitungen einer Subjektseite des Pflasters zugewandt ist, wobei das Pflaster ein dehnbares Substrat beinhaltet, das in die Vielzahl von dehnbaren Leitungen integriert ist und einen Schaltungsbereich beinhaltet, in dem sich Schaltung aufhält; und
Verwenden der Schaltung, um die elektrischen Signale, die entlang jeder aus der Vielzahl von dehnbaren Leitungen gegeben werden, zu sammeln.

15. Verfahren nach Anspruch 14, ferner beinhaltend Verwenden der Schaltung, um verschiedene der elektrischen Signale als Reaktion auf eine konfigurierbare und/oder programmierbare Benutzereinstellung zu verstärken, wobei das dehnbare Polymer elastische Eigenschaften aufweist.

16. Vorrichtung nach Anspruch 13, beinhaltend eine Steuerung, die konfigurierbare oder programmierbare Logikschaltung aufweist, und ferner Folgendes beinhaltet: eine Vielzahl von Mehrkanalverstärkern, wobei jeder der Mehrkanalverstärker gemäß zumindest einem des Folgenden konfiguriert ist: in der Lage zu sein, selektiv eines der elektrischen Signale als Reaktion auf Selektivitätssteuerung, die durch die Steuerung bereitgestellt ist, zu verstärken, und ein Paar von Eingängen aufweisend, die als eine EKG-Leitung konfiguriert sind.

17. Vorrichtung nach Anspruch 13, ferner beinhaltend ein Pflaster, das mit der Vielzahl von Elektroden assoziiert ist, wobei die Vielzahl von Elektroden vier bis neun einzelne Elektroden zur Platzierung gegen das Subjekt beinhaltet und wobei das Pflaster weniger als sieben Quadratzoll von Hautbereich des Subjekts einnehmen soll, während es an dem Subjekt getragen wird.

18. Vorrichtung nach Anspruch 13, ferner beinhaltend Schaltung (160, 260), um die elektrischen Signale zu konditionieren und zu verstärken, um eines oder mehrere von Folgendem zu erfassen: einer Verschiebung in elektrischer Achse, breiten QRS-Komplexen und einer Herzarrhythmie.

19. Vorrichtung nach Anspruch 13, wobei die Vielzahl von Elektroden mit einer Vielzahl von dehnbaren Leitungen und einem Pflaster assoziiert ist, das Folgendes beinhaltet: eine erste Materialschicht, die das dehnbare Substrat (110, 210) aufweist, eine zweite Materialschicht (110a, 210a) auf einer Seite der ersten Materialschicht und die Vielzahl von dehnbaren Leitungen aufweisend, und zumindest eine Ausnehmung (120, 220), die durch die erste Materialschicht gebildet ist, mit einem Leiter, der die zumindest eine Ausnehmung überlappt, wobei sich eine Zwischenverbindung (140, 240) auf einer anderen gegenüberliegenden Seite der ersten Materialschicht befinden soll und an zumindest einer von der Sensorschaltung und der Prozessorschaltung zu sichern ist, wobei die zumindest eine Ausnehmung als ein Dielektrikum und ein Pfadweg für den Leiter dienen soll, um die elektrischen Signale von der einen Seite zu der anderen Seite der ersten Materialschicht zu transportieren.

## Revendications

1. Appareil comprenant :
une pluralité de fils étirables, chacun de la pluralité de fils étirables comprenant un polymère étirable et une électrode associée qui doit recevoir des signaux électriques générés en réponse au cœur d'un patient et faire passer les signaux électriques reçus le long d'un fil respectif de la pluralité de fils étirables ; et
un patch comprenant un substrat étirable (110, 210) intégré à la pluralité de fils étirables et comprenant une zone d'ensemble de circuits permettant à l'ensemble de circuits (160, 260) de résider pour collecter des signaux électriques passés le long de chaque fil de la pluralité de fils étirables, chaque fil de la pluralité de fils étirables devant être sur un côté patient du patch.

2. Appareil de la revendication 1, comprenant en outre l'ensemble de circuits (160, 260), et ledit ensemble de circuits devant être fixé au niveau de la zone d'ensemble de circuits du patch et couplé à la pluralité de fils étirables, et ledit polymère étirable comportant des propriétés élastiques.

3. Appareil de la revendication 1, ledit substrat étirable (110, 210) comportant au moins un orifice (120, 220) formé dans celui-ci et comportant en outre un conducteur chevauchant ledit au moins un orifice, ledit conducteur chevauchant ledit au moins un orifice devant faire passer les signaux électriques reçus de la pluralité de fils étirables le long d'un trajet menant à la zone d'ensemble de circuits.

4. Appareil de la revendication 1, comprenant en outre l'ensemble de circuits (160, 260) fixé au niveau de la zone d'ensemble de circuits, et ledit ensemble de circuits comprenant un dispositif de commande comportant un ensemble de circuits logique configurable ou programmable et comprenant en outre : une pluralité d'amplificateurs multicanaux, chacun des amplificateurs multicanaux étant configuré selon au moins l'un des suivants : être capable d'amplifier sélectivement l'un des signaux électriques en réponse à une commande de sélectivité fournie par le dispositif de commande, et comporter une paire d'entrées en tant que fil ECG.

5. Appareil de la revendication 1, ladite pluralité d'électrodes comprenant quatre à neuf électrodes individuelles destinées à être placées contre le patient par l'intermédiaire du patch, et ledit patch devant occuper moins de sept pouces carrés de la zone de peau du patient lorsqu'il est porté sur le patient.

6. Appareil de la revendication 1, ledit ensemble de circuits (160, 260) devant conditionner et amplifier les signaux électriques pour détecter un ou plusieurs parmi : un décalage d'axe électrique, des complexes QRS larges et une arythmie cardiaque.

7. Appareil de la revendication 1, ledit patch comprenant une première couche de matériau comportant le substrat étirable (110, 210), une seconde couche de matériau (110a, 210a) sur un côté de la première couche de matériau et comportant la pluralité de fils étirables, et au moins un orifice (120, 220) formé à travers la première couche de matériau doté d'un conducteur chevauchant ledit au moins un orifice, une interconnexion (140, 240) devant être située sur un autre côté opposé de la première couche de matériau et devant être fixée à l'ensemble de circuits (160, 260) au niveau de la zone d'ensemble de circuits.

8. Appareil de la revendication 7, ledit au moins un orifice (120, 220) devant agir en tant que diélectrique et trajet pour le conducteur pour transporter les signaux électriques d'un côté à l'autre de la première couche de matériau.

9. Appareil de la revendication 7, ledit substrat (110, 210) étant formé par coulage par goutte sous la forme d'un film solide, ladite pluralité d'électrodes et ladite pluralité de fils étirables étant de l'encre conductrice sérigraphiée, et ledit ensemble de circuits comprenant de l'encre conductrice sérigraphiée sous la forme de traces pour l'ensemble de circuits sur l'autre côté opposé de la première couche de matériau.

10. Appareil de la revendication 1, comprenant en outre :
l'ensemble de circuits (160, 260), et ledit ensemble de circuits devant être fixé au niveau de la zone d'ensemble de circuits du patch et couplé à la pluralité de fils étirables ; et
un circuit de collecte de données et de traitement de signaux, comprenant un circuit récepteur pour recevoir des signaux conditionnés dérivés des signaux électriques et comprenant un circuit de mémoire pour stocker les signaux conditionnés.

11. Appareil de la revendication 10, ledit circuit de collecte de données et de traitement de signal comprenant ou faisant partie d'un circuit informatique mis en œuvre avec une interface utilisateur et configuré pour fournir des informations associées à un ECG.

12. Appareil de la revendication 1, des adhésifs conducteurs étant fixés aux électrodes, tandis que des adhésifs isolants sont fixés à des zones orthogonales aux électrodes sur le substrat.

13. Appareil comprenant :
un ensemble de circuits de détection comprenant :
un substrat étirable comportant un orifice (120, 220) formé dans celui-ci ;
un premier substrat conducteur et étirable (110, 210) comprenant une pluralité d'électrodes agencées en un réseau et des premières interconnexions (140, 240) qui couplent la pluralité d'électrodes à une première ouverture de l'orifice, la pluralité d'électrodes étant configurées et agencées pour entrer en contact avec un patient et/ou pour capturer des signaux électriques émis par un cœur du patient ;
un second substrat conducteur et étirable (110a, 210a) comprenant des secondes interconnexions (130, 230) qui se couplent à une seconde ouverture de l'orifice, l'orifice pour fournir une connexion électrique entre la pluralité d'électrodes, les premières interconnexions et les secondes interconnexions ; et
un matériau adhésif couplé au premier substrat conducteur et étirable, le matériau adhésif étant configuré et agencé pour coupler l'appareil de détection au patient ; et
un ensemble de circuits de processeur configuré et agencé avec l'ensemble de circuits de détection pour obtenir les signaux électriques tels que capturés par la pluralité d'électrodes et pour fournir un ECG à conducteurs multiples sur la base des signaux électriques.

14. Procédé comprenant :
la réception des signaux électriques générés en réponse au cœur d'un patient à l'aide d'électrodes respectivement associées à une pluralité de fils étirables, chacun comprenant un polymère étirable, dans le cadre d'un patch comportant un substrat étirable (110, 210), et lesdits signaux électriques reçus étant passés le long d'un fil respectif de la pluralité de fils étirables tandis que chaque fil de la pluralité de fils étirables fait face à un côté patient
du patch, ledit patch comprenant un substrat étirable intégré à la pluralité de fils étirables et comprenant une zone d'ensemble de circuits permettant à l'ensemble de circuits de résider ; et l'utilisation de l'ensemble de circuits pour collecter les signaux électriques passés le long de chaque fil de la pluralité de fils étirables.

15. Procédé de la revendication 14, comprenant en outre l'utilisation de l'ensemble de circuits pour amplifier différents signaux électriques parmi les signaux électriques en réponse à un réglage utilisateur configurable et/ou programmable, ledit polymère étirable comportant des propriétés élastiques.

16. Appareil de la revendication 13, comprenant un dispositif de commande comportant un ensemble de circuits logique configurable ou programmable et comprenant en outre : une pluralité d'amplificateurs multicanaux, chacun des amplificateurs multicanaux étant configuré selon au moins l'un des suivants : être capable d'amplifier sélectivement l'un des signaux électriques en réponse à une commande de sélectivité fournie par le dispositif de commande, et comporter une paire d'entrées configurées en tant que fil ECG.

17. Appareil de la revendication 13, comprenant en outre un patch associé à la pluralité d'électrodes, ladite pluralité d'électrodes comprenant de quatre à neuf électrodes individuelles destinées à être placées contre le patient et ledit patch devant occuper moins de sept pouces carrés de la zone de peau du patient lorsqu'il est porté sur le patient.

18. Appareil de la revendication 13, comprenant en outre un ensemble de circuits (160, 260) pour conditionner et amplifier les signaux électriques afin de détecter un ou plusieurs parmi : un décalage d'axe électrique, des complexes QRS larges et une arythmie cardiaque.

19. Appareil de la revendication 13, ladite pluralité d'électrodes étant associées à une pluralité de fils étirables et à un patch qui comprend : une première couche de matériau comportant le substrat étirable (110, 210), une seconde couche de matériau (110a, 210a) sur un côté de la première couche de matériau et comportant la pluralité de fils étirables, et au moins un orifice (120, 220) formé à travers la première couche de matériau doté d'un conducteur chevauchant ledit au moins un orifice, une interconnexion (140, 240) devant être située sur un autre côté opposé de la première couche de matériau et devant être fixée à au moins l'un de l'ensemble de circuits de détection et de l'ensemble de circuits de processeur, ledit au moins un orifice devant agir en tant que diélectrique et trajet pour le conducteur afin de transporter les signaux électriques d'un côté à l'autre de la première couche de matériau.
